# EUROPEAN PATENT SPECIFICATION

(11) **EP 1 521 535 B1**
(45) Date of publication and mention of the grant of the patent: **01.02.2006**
(21) Application number: 03738345.2
(22) Date of filing: 11.07.2003
(51) Int. Cl.: A23L 1/29, A23L 1/308, A61K 9/14

(54) **IMPROVED BULKING AGENT COMPOSITION**
VERBESSERTE FÜLLMITTELZUSAMMENSETZUNGEN
COMPOSITIONS AMELIOREES D'AGENTS GONFLANTS

(30) Priority: 12.07.2002 GB 0216147
(43) Date of publication of application: 13.04.2005
(73) Proprietor: Reckitt Benckiser Healthcare (UK) Limited, Slough, Berkshire SL1 3UH (GB)
(72) Inventor: DETTMAR, Peter, William, Patrington, East Yorkshire HU12 0PE (GB); HAVLER, Michael, E., Hull HU8 7DS (GB); MCKIE, Kevin, Hessle, East Yorkshire HU13 9HR (GB)
(74) Representative: Brown, Andrew Stephen
(86) International application number: PCT/GB2003/003040
(87) International publication number: WO 2004/006692

(56) References cited:
- EP-A- 0 317 659
- EP-A- 0 368 534
- EP-A- 0 583 852
- WO-A-80/00658
- US-A- 5 370 892
- US-B1- 6 214 349

## Description

The present invention relates to medicinal compositions comprising fibre bulking agents.

Ingestible fibre- compositions for the relief of gastric and digestive dysfunctions are known. EP-A-0583852 describes a composition containing polysaccharides (bran, fiber, oligosaccharides, cellulose), surfactant and silicate. The product is easily dispersible. Other compositions include granular psyllium husk fibre (ispaghula) intended to be stirred in measured amounts into a volume of liquid, usually water or soft drinks. After stirring, the drinking composition is intended to be quickly imbibed due to the propensity of the ispaghula to absorb water readily and swell to form a viscous gel-like mass. It is the property of water absorption which has the desired characteristic of fibre or saccharide-containing ingestible compositions for gastric and digestive dysfunctions. Once the fibre or saccharide-containing composition has absorbed water to produce the gel-like mass, the mass is relatively insoluble and fibrous, and is transported through the gut quickly with minimal digestion, helping to alleviate constipation and other digestive dysfunctions.

Other forms, such as capsules forms for ingestion, are also available, such capsules being designed to be broken down in the gut, wherein the released fibre or saccharide bulking agent absorbs water from the gut to form the viscous mass.

However, for beneficial ease-of-use properties, a particulate form is particularly advantageous to the end user, as this can be stirred into a volume of liquid, for a more pleasant taste, and the granular form of the fibre absorbs water from the gut more quickly than a capsule form. However, there are a number of problems involved in using a granular form of the fibre-containing ingestible compositions.

Primarily, it is desirable for the ingestible compositions to disperse easily in liquid, for the user's convenience and/or so that the resultant drink is more palatable and/or easier to swallow. Any new composition must be as good as or, preferably, better than, existing compositions in this respect.

Secondly, the handling of some ingestible fibre-containing compositions is not straightforward. For example in commercial production ispaghula is milled then isopropyl alcohol and a granulating agent polyvinyl pyrollidone are added. These steps aid handling of the compositions during manufacturing, before the isopropyl alcohol is removed prior to packaging the product for sale. The granulation also aids the dispersion of the ispaghula into a volume of liquid, prior to ingestion. However, the use of the granulating agent and isopropyl alcohol increases the cost of production and the use of the isopropyl alcohol is undesirable from an environmental and a health and safety perspective.

Thus, from the foregoing, it is apparent that there is a need for the provision of an ingestible composition which comprises a fibre bulking agent, in which the ingestible composition disperses easily in an aqueous liquid and/or is of improved manufacture.

It has now been determined that an ingestible composition comprising a psyllium husk fibre bulking agent (ispaghula), colloidal silica in conjunction with an ingestible surfactant, can offer benefit in the manufacture of the ingestible composition, and can increase the rate at which the ingestible composition disperses in water or other ingestible liquid.

Therefore, according to the present invention there is provided an ingestible composition comprising ispaghula, colloidal silica and an ingestible surfactant wherein said composition is in a form so that in use it is dispersed in a liquid prior to ingestion.

The presence of both an ingestible silica and an ingestible surfactant can confer significant, eg synergistic, benefits. For example, the ternary composition of the ispaghula has outstanding wettability properties, and is easy to manufacture, for example by simple blending.

Suitably the fibre bulking agent is a natural ingestible fibre (by which term we include herein fibre extracts). Plant-derived fibre bulking agents from psyllium husk fibre (ispaghula) are used.

The ispaghula may comprise whole ispaghula seeds, but preferably at least part of the ispaghula comprises separated ispaghula seed husks. More preferably the ispaghul a comprises at least 50% wt separated ispaghula husks, most preferably at least 95% wt separated ispaghula husks. Suitably the remainder of the ispaghula comprises other seed parts and/or other ispaghula plant materials. In preferred compositions the seed kernels themselves have been substantially removed to leave the husks. Suitably the fibre bulking agent is present in the ingestible composition in an amount of at least 10wt%, preferably at least 30wt%, and most preferably at least 40wt% of the total weight of the ingestible composition.

Suitably the fibre bulking agent is present in the ingestible composition in an amount up to 90wt%, preferably up to 80wt%, and most preferably up to 75wt% of the total weight of the ingestible composition.

Suitably the colloidal silica is fumed or precipitated synthetic or natural silica. The silica may be amorphous or crystalline.

Suitably the mean particle size of the silica is at least 5nm, preferably at least 10nm.

Suitably the mean particle size of the silica is up to 5µm, preferably up to 0.75µm, more preferably up to 0.5µm, and most preferably up to 0.2µm.

One suitable silica material is Syloid 244 which is amorphous silica, has a mean particle size of about 3µm and is provided by W R Grace & Co. Another suitable silica materials is Silox 15,also from W R Grace & Co., and which has a mean particle size of about 4µm.

Another suitable silica material is Huber Zep 49 which is amorphous silica from J M Huber Corporation and contains about 1 wt% alumina.

Another suitable silica is Aerosil 200 from Degussa Company. It contains less than 0.05 wt% alumina and has a mean particle size of 12 nm.

The silica is colloidal silica (silicon dioxide), and a preferred silica is a colloidal silica which is sold under the trade mark CAB-O-SIL, by Cabot Inc, USA.

Suitably the specific surface area of the silica is at least 50m² g⁻¹, preferably at least 150m² g⁻¹.

Suitably the specific surface area of the silica is up to 400m² g⁻¹, preferably up to 300m² g⁻¹ most preferably up to 200m² g⁻¹.

Suitably the silica is present in the ingestible composition in an amount at least 0.01wt%, preferably at least 0.05wt%, more preferably at least 0.1wt% and most preferably at least 0.25wt%, of the total weight of the ingestible composition.

The upper limit of silica in the ingestible composition may be up to 11 wt%. Suitably the silica may be present in the ingestible composition in an amount up to 5wt%, preferably up to 2wt%, more preferably up to 1wt%, and most preferably up to 0.6wt%, of the total weight of the ingestible composition.

Preferably the ingestible surfactant is a polyethylene-, polypropylene-, or polyoxyethylene-based surfactant. Suitable polyethylene or polyoxyethylene-based surfactants include polyethylene glycols and polyoxyethylene sorbitan fatty acid esters (polysorbates).

Suitable polyethylene glycols have a molecular weight of between 200 and 40,000, preferably between 200 and 1,000, and more preferably between 200 and 600. Suitable polyethylene glycols include MACROGOLD and MACROGOLUM polyethylene glycols sold by ICI Surfactants, UK. Other suitable surfactants include polyoxyethylene monostearates and glycerol polyethylene glycol oxystearates.

Suitably the surfactant is present in the ingestible composition in an amount at least 0.01wt%, preferably at least 0.05wt%, more preferably at least 0.1wt%, and most preferably at least 0.2wt%, of the total weight of the ingestible composition.

Suitably the surfactant is present in the ingestible composition in an amount up to 5wt%, preferably up to 3wt%, more preferably up to 2wt% and most preferably up to 1wt%, of the total weight of the ingestible composition.

When the surfactant is polyethylene glycol it is preferably present is an amount at least 0.1wt%, more preferably at least 0.3wt%, of the total weight of the ingestible composition.

When the surfactant is polyethylene glycol it is preferably present is an amount up to 2wt%, more preferably up to 1.5wt%, of the total weight of the ingestible composition.

When the surfactant is a polyoxyethylene sorbitan fatty acid ester it is preferably present in an amount at least 0.01wt%, more preferably at least 0.05wt%, and most preferably at least 0.08wt%, of the total weight of the ingestible composition.

When the surfactant is a polyoxyethylene sorbitan fatty acid ester it is preferably present in an amount up to 2wt%, more preferably up to 1wt%, and most preferably up to 0.5wt%, of the total weight of the ingestible composition.

The percentages stated represent the total complement of the silica and surfactant, that is, summated if there is more than one silica or surfactant in the composition.

The ingestible composition may further comprise ingestible co-ingredients such as a bicarbonate for example sodium bicarbonate, an ingestible acid, for example citric acid, a flavouring, or a colouring, for example.

Preferably the ingestible composition does not contain a granulating agent.

Most preferably the ingestible composition does not contain polyvinyl pyrollidone.

Preferably the ingestible composition does not contain any residue of polyvinyl alcohol.

The composition is particularly preferred in a form such that it is easily dispersed in a liquid such as water before drinking. Suitably the composition is provided in a particulate or granular solid form, for example as a powder or flakes, intended to be mixed with water, prior to ingestion by a user. Alternatively the composition may be provided as a capsule for dispersal in a liquid, for drinking by a user. Preferably the composition is provided in a particulate form.

In accordance with a second aspect of the present invention there is provided a method of making an ingestible composition comprising a fibre or saccharide bulking agent, an ingestible silica, and an ingestible surfactant, the method comprising the step of blending the fibre or saccharide bulking agent with the ingestible silica and the ingestible surfactant.

Preferably no isopropyl alcohol is used in the manufacture.

More preferably no solvent of any type is used in the manufacture.

Preferably no polyvinyl pyrollidone is used in the manufacture.

More preferably no granulating agent of any type is used in the manufacture.

The fibre or saccharide bulking agent may be milled prior to the blending step, suitably to a mean particle size in the range 250-450 µm.

Preferably the method does not include the granulation of the bulking agent.

The fibre or saccharide bulking agent may be subjected to a sterilization step prior to the blending step. Irradiation may employ steam or, preferably, a radioactive source, for example a γ-radiation source, for example from a Cobalt-60 or Caesium-137 source. A suitable radiation dosage is up to 13 kGy, preferably 5-10 kGy.

The invention will now be described by way of example in which the following materials are used throughout:
Ispaghula - Ispaghula husk material obtained from Plantago ovata, broken down to enable the seed kernels to be removed. The material was dried, irradiated with γ-radiation from a Caesium-137 source at a dosage rate of about 7 kGy, as described in PCT/GB01/02040, and milled to a mean particle size of 300-400µm.
CAB-O-SIL (Trade Mark) - A colloidal silica having a specific surface area in the range 175-225m²g⁻¹ manufactured by Cabot Inc, USA.
TWEEN 60 (Trade Mark) - A polyoxyethylene sorbitan fatty acid ester, manufactured by ICI.
TWEEN 80 (Trade Mark) - A polyoxyethylene sorbitan fatty acid ester, manufactured by ICI.
Propylene glycol.
Cremophore RH40 - Glycerol polyethylene glycol oxysterate, manufactured by BASF.
Pricerine - Porcine glyerine, supplied by Uniquema.
PEG 200 - Polyethylene glycol, molecular weight approximately 200, manufactured by Clariant.
Liquid surfactants were added slowly to the ispaghula as it was being mixed in a domestic-style MAGIMIXER (Trade Mark). Mixing was continued until the ispaghula appeared evenly covered (dampened).
Solid surfactants were ground in a pestle and mortar and then added to the ispaghula and placed in an oven (60-70°C) for 30 minutes. The samples were then quickly blended in the MAGIMIXER as above.

To the ispaghula blended with surfactant as described above was added a colloidal silica sample (CAB-O-SIL) in an attempt to dust the samples dry and improve flow characteristics (but also - as will be seen - with the unexpected result that the wetting characteristics of the final product were greatly improved).

No granulation step took place; no granulating agent was used.

### Test methods

### 1. Wettability (dispersion in water)

This was the most important test used to assess the effectiveness of each treatment and simply involved slowly spreading an amount of the formulation containing 3.5g ispaghula onto the surface of 150ml of cold tap water contained in a 200ml Pyrex beaker, and recording the time taken for all the material to become fully wetted without using any agitation to quicken the process.

### 2. Water absorbency/swell volume

This test determines the swell volume (ml) or ability of a product to take up water. This is a key property for the mechanism of action of ispaghula, and hence disruption/reduction to this effect would certainly impact on efficiency.

### The method is as follows:

Add 1g of ispaghula (or the equivalent wt of product containing 1g ispaghula) to 100ml of tap water in a 100ml measuring cylinder, mix thoroughly by shaking and allow to stand. At 1 and 2 hours mix again by gentle inversion, and allow to stand for a further 2 hours. At the end of this period (4 hours from start), record the level of mucilage/gel in the measuring cylinder. Typically, this will be 40-50ml per gramme of ispaghula.

### 3. Gel/flow rate on hydration

This method is used to gain an insight into the rate of gel/mucilage formation. Although gelling is an important attribute, the initial onset has been delayed in ingestible ispaghula compositions to allow the consumer to ingest it, over a period of a few minutes, as a palatable drink.

A weight of sample containing 3.5g ispaghula is mixed into 150ml cold tap water in a 200ml beaker. At 5 minute intervals after making up, the time taken for 100ml of the sample to run through a Flow Cup (No. 5) viscometer is recorded. This is basically a brass cup which holds exactly 100ml, with a tapered bottom leading to a standard-sized hole. As a sample gels, then the time taken for 100ml to flow through increases.

### 4. Carr's Index

Carr's index is a measurement of bulk density of pharmaceutical powders, measured in a Copley Erweka Tapped Volumeter, model SVM-22. Powder is placed in a vertical cylinder which is "tapped" in the machine to aid the settlement of the powder, and the percentage change in volume measured, over the predetermined test period/regime, identical for each sample.

### Example 1

In this series of tests the wetting ability of ternary ispaghula + PEG 200 + CAB-O-SIL compositions was assessed, and compared with non-ternary compositions. The wettability was measured after 5 minutes, 16-24 hours and 8 days; there is reason from work on other compositions to believe that wettability can decrease as the interval from manufacture increases.

As will be seen, there were three replicates. All three results are given in Tables 1-3 below.

In each test the measurement was of time (secs) for a dose of treated ispaghula (3.5g) to disperse.

### Tables 1-3

**PEG 200 + CAB-O-SIL 5 mins after manufacture**

| | **CAB-O-SIL levels (wt%)** | |
|---|---|---|
| **PEG 200 levels (wt%)** | **0.3** | **0.5** |
| **0.4** | **5,5,5** | **7,5,6** |
| **0.8** | **7,8,7** | **6,5,5** |
| **1.2** | **6,4,4** | **6,7,7** |

**Comparisons: no PEG 200, no CAB-O-SIL: 270, 310, 330 no PEG 200, 0.3 wt% CAB-O-SIL: 165, 170, 165 0.4 wt% PEG 200, no CAB-O-SIL: 120, 130, 135**

**PEG 200 + CAB-O-SIL 16-24 hours after manufacture**

| | **CAB-O-SIL levels (wt%)** | |
|---|---|---|
| **PEG 200 levels (wt%)** | **0.3** | **0.5** |
| **0.4** | **6,6,6** | **7,5,7** |
| **0.8** | **20,20,20** | **6,5,6** |
| **1.2** | **11,12,11** | **12,11,12** |

**Comparisons: no PEG 200, no CAB-O-SIL: not measured no PEG 200, 0.3 wt% CAB-O-SIL: 330, 330, 350 0.4 wt% PEG 200, no CAB-O-SIL: 305, 320, 320**

**PEG 200 + CAB-O-SIL 8 days after manufacture**

| | **CAB-O-SIL levels (wt%)** | |
|---|---|---|
| **PEG 200 levels (wt%)** | **0.3** | **0.5** |
| **0.4** | **11,12,11** | **10,6,7** |
| **0.8** | **25,25,30** | **8,8,8** |
| **1.2** | **22,30,26** | **15,17,15** |

**Comparisons: no PEG 200, no CAB-O-SIL: 310, 300, 280 no PEG 200, 0.3 wt% CAB-O-SIL: 1800, 1200, 1740 0.4 wt% PEG 200, no CAB-O-SIL : 470, 480, 510.**

### Example 2

This series of tests were as Example 1, but used TWEEN 80 instead of PEG 200, and different time intervals. In these tests ispaghula alone was not tested. The results are given in Tables 4-6 below. Again, in each test the measurement was of time (secs) for a dose of treated ispaghula (3.5g) to disperse (n=3).

### Tables 4-6

**TWEEN 80 + CAB-O-SIL 5 mins after manufacture**

| | **CAB-O-SIL levels (wt%)** | |
|---|---|---|
| **TWEEN 80 levels (wt%)** | **0.3** | **0.5** |
| **0.09** | **21,16,17** | **20,17,20** |
| **0.14** | **7,7,8** | **8,5,8** |
| **0.20** | **4,4,5** | **4,4,4** |

**Comparisons: no TWEEN 80, no CAB-O-SIL: not measured no TWEEN 80, 0.3 wt% CAB-O-SIL: 180, 215, 225 0.09wt% TWEEN 80, no CAB-O-SIL : 75, 80, 75**

**TWEEN 80 + CAB-O-SIL 72 hours after manufacture**

| | **CAB-O-SIL levels (wt%)** | |
|---|---|---|
| **TWEEN 80 levels (wt%)** | **0.3** | **0.5** |
| **0.09** | **25,25,30** | **30,22,25** |
| **0.14** | **10,13,11** | **10,10,9** |
| **0.20** | **6,5,4** | **4,5,5** |

**Comparisons: no TWEEN 80, no CAB-O-SIL: not measured no TWEEN 80, 0.3 wt% CAB-O-SIL: 495, 450, 480 0.09wt% TWEEN 80, no CAB-O-SIL : 63, 70, 60**

**TWEEN 80 + CAB-O-SIL 7 days after manufacture**

| | **CAB-O-SIL levels (wt%)** | |
|---|---|---|
| **TWEEN 80 levels (wt%)** | **0.3** | **0.5** |
| **0.09** | **36,27,32** | **35,35,35** |
| **0.14** | **10,10,12** | **10,9,8** |
| **0.20** | **7,7,6** | **4,5,5** |

**Comparisons: no TWEEN 80, no CAB-O-SIL: not measured no TWEEN 80, 0.3 wt% CAB-O-SIL: 735, 795, 930 0.09wt% TWEEN 80, no CAB-O-SIL : 75, 67, 78**

Again the results for the ternary system are remarkable, much better than either binary system.

### Example 3

This test was used primarily to assess long term wettability properties. Obviously such properties are extremely important for a commercial product.

As before, each experiment employed 3.5g of ispaghula in the composition, except that for the swell volume test 1g of composition was used.

The compositions were placed in a cycling oven, cycling between 4°C and 30°C. The samples were tested immediately on preparation, after 5 weeks incubation in the cycling oven, and after 3 months incubation the cycling oven. As an exception, compositions including CAB-O-SIL and PEG 200 were tested immediately and after 9 weeks incubation only. The results of the experiment are shown in Table 7.

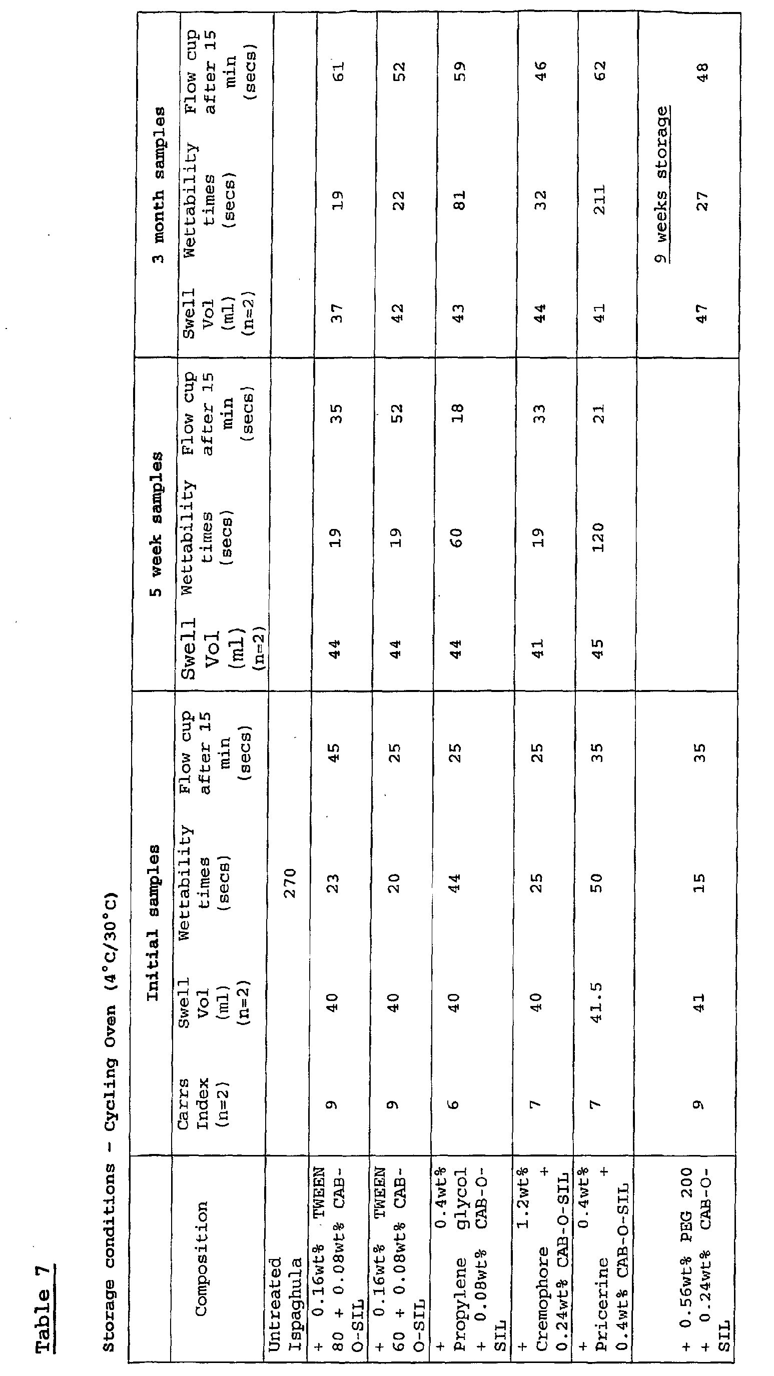

The results show that the addition of all of the tested combinations of ingestible silica in combination with an ingestible surfactant, substantially reduces the time taken for the ispaghula to disperse in a 150ml beaker of cold water (wettability). The wettability time is significantly reduced on initial testing and remains reduced through the 5 week samples and the 3 month samples.

In particular, polyethylene glycol and TWEEN in combination with CAB-O-SIL show a marked ability to reduce the wettability time of untreated ispaghula husk compared to other combinations of surfactant with CAB-O-SIL. The results therefore indicate that ingestible compositions comprising surfactant plus CAB-O-SIL mixed with ispaghula husk are also shelf stable at ambient temperatures (between 4°C and 30°C) over a sustained period of time.

### Example 4

The tests corresponded to those of Example 3 but the compositions were tested by incubating at 40°C in an incubating oven. The samples were tested immediately on preparation, after 5 weeks incubation and after 3 months incubation. As an exception, compositions containing CAB-O-SIL and PEG 200 were tested immediately and after 9 weeks incubation only. The results of the experiment are shown in Table 8.

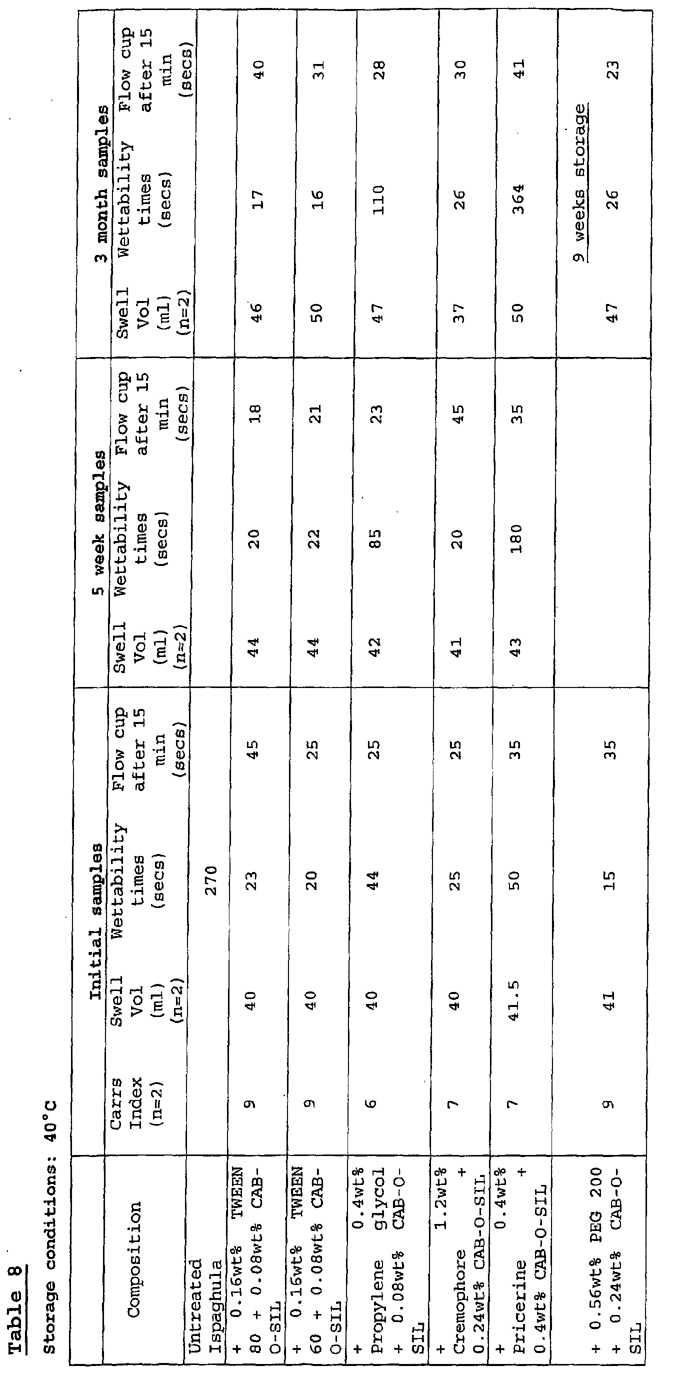

## Claims

1. An ingestible composition comprising ispaghula, colloidal silica, and an ingestible surfactant wherein said composition is in a form so that in use it is dispersed in a liquid prior to ingestion.

2. An ingestible composition according to claim 1 wherein said composition in particulate or granular form.

3. An ingestible composition as claimed in any preceding claim wherein the particle size of the silica is between 5nm and 5µm.

4. An ingestible composition as claimed in any preceding claim wherein the specific surface area of the silica is between 50 and 400gm⁻².

5. An ingestible composition as claimed in any preceding claim wherein the silica is present in an amount of between 0.01wt% and 5wt% of the total weight of the ingestible composition.

6. An ingestible composition as claimed in any preceding claim, wherein the ingestible surfactant is a polyethylene-, polypropylene-, or polyoxyethylene-based surfactant.

7. An ingestible composition as claimed in claim 6 wherein the polyethylene-based surfactant is a polyethylene glycol.

8. An ingestible composition as claimed in claim 7 wherein the polyethylene glycol has a molecular weight of between 200 and 40,000, preferably between 200 and 1,000.

9. An ingestible composition as claimed in claim 6 wherein the polyoxyethylene-based surfactant is a polyoxyethylene sorbitan fatty acid eater.

10. An ingestible composition as claimed in claim 6 wherein the surfactant is a polyoxyethylene monostearate or a glycerol polyethylene glycol oxyetearate.

11. An ingestible composition as claimed in any preceding claim wherein the ingestible surfactant is present in an amount of between 0.01wt% and 5wt% of the total weight of the ingestible composition.

12. An ingestible composition as claimed in claim 11 wherein the ingestible surfactant is polyethylene glycol and is present in an amount of between 0.1wt% and 2wt% of the total weight of the ingestible composition.

13. An ingestible composition as claimed in claim 11 wherein the surfactant is a polyoxyethylene sorbitan fatty acid ester and is present in an amount of between 1wt% and 2wt% of the total weight of the ingestible composition.

14. A method of making an ingestible composition comprising ispaghula, colloidal silica, and an ingestible surfactant, the method comprising the step of blending the ispaghula with the colloidal silica and the ingestible surfactant; preferably without the employment of isopropyl alcohol or more preferably of any solvent; and preferably without the employment of polyvinyl pyrollidone or more preferably of any granulating agent.

## Patentansprüche

1. Einnehmbare Zusammensetzung, umfassend Ispaghula, kolloidales Siliciumdioxid und ein einnehmbares oberflächenaktives Mittel, wobei die Zusammensetzung in einer derartigen Form vorliegt, dass sie vor der Einnahme in einer Flüssigkeit dispergiert wird.

2. Einnehmbare Zusammensetzung nach Anspruch 1, wobei die Zusammensetzung in Teilchen- oder Granulatform vorliegt.

3. Einnehmbare Zusammensetzung nach einem der vorangehenden Ansprüche, wobei die Teilchengröße des Siliciumdioxids zwischen 5 nm und 5 µm liegt.

4. Einnehmbare Zusammensetzung nach einem der vorangehenden Ansprüche, wobei die spezifische Oberfläche des Siliciumdioxids zwischen 50 und 400 gm⁻² liegt.

5. Einnehmbare Zusammensetzung nach einem der vorangehenden Ansprüche, wobei das Siliciumdioxid in einer Menge zwischen 0,01 Gew.-% und 5 Gew.- % des Gesamtgewichts der einnehmbaren Zusammensetzung vorliegt.

6. Einnehmbare Zusammensetzung nach einem der vorangehenden Ansprüche, wobei das einnehmbare oberflächenaktive Mittel ein oberflächenaktives Mittel auf Polyethylen-, Polypropylen- oder Polyoxyethylen-Basis ist.

7. Einnehmbare Zusammensetzung nach Anspruch 6, wobei das oberflächenaktive Mittel auf Polyethylen-Basis ein Polyethylenglycol ist.

8. Einnehmbare Zusammensetzung nach Anspruch 7, wobei das Polyethylenglycol ein Molekulargewicht zwischen 200 und 40.000, vorzugsweise zwischen 200 und 1.000 aufweist.

9. Einnehmbare Zusammensetzung nach Anspruch 6, wobei das oberflächenaktive Mittel auf Polyoxyethylen-Basis ein Polyoxyethylensorbitanfettsäureester ist.

10. Einnehmbare Zusammensetzung nach Anspruch 6, wobei das oberflächenaktive Mittel ein Polyoxyethylenmonostearat oder ein Glycerinpolyethylenglycoloxystearat ist.

11. Einnehmbare Zusammensetzung nach einem der vorangehenden Ansprüche, wobei das einnehmbare oberflächenaktive Mittel in einer Menge zwischen 0,01 Gew.% und 5 Gew.-% des Gesamtgewichts der einnehmbaren Zusammensetzung vorliegt.

12. Einnehmbare Zusammensetzung nach Anspruch 11, wobei das einnehmbare oberflächenaktive Mittel Polyethylenglycol ist und in einer Menge zwischen 0,1 Gew.-% und 2 Gew.-% des Gesamtgewichts der einnehmbaren Zusammensetzung vorliegt.

13. Einnehmbare Zusammensetzung nach Anspruch 11, wobei das oberflächenaktive Mittel ein Polyoxyethylensorbitanfettsäureester ist und in einer Menge zwischen 1 Gew.-% und 2 Gew.-% des Gesamtgewichts der einnehmbaren Zusammensetzung vorliegt.

14. Verfahren zur Herstellung einer einnehmbaren Zusammensetzung, umfassend Ispaghula, kolloidales Siliciumdioxid und ein einnehmbares oberflächenaktives Mittel, wobei das Verfahren den Schritt des Mischens des Ispaghulas mit dem kolloidalen Siliciumdioxid und dem einnehmbaren oberflächenaktiven Mittel vorzugsweise ohne Einsatz von Isopropylalkohol oder stärker bevorzugt jeglichen Lösungsmittels und vorzugsweise ohne Einsatz von Polyvinylpyrrolidon oder stärker bevorzugt jeglichen Granulierungsmittels umfasst.

## Revendications

1. Composition ingestible comprenant de l'ispaghula, de la silice colloïdale et un agent tensio-actif ingestible, ladite composition étant sous une forme convenant pour qu'à l'emploi elle soit dispersée dans un liquide avant l'ingestion.

2. Composition ingestible selon la revendication 1, dans laquelle ladite composition est sous forme particulaire ou granulaire.

3. Composition ingestible selon l'une quelconque des revendications précédentes, dans laquelle la taille des particules de la silice est comprise entre 5 nm et 5 µm.

4. Composition ingestible selon l'une quelconque des revendications précédentes, dans laquelle la surface spécifique de la silice est comprise entre 50 et 400 gm⁻².

5. Composition ingestible selon l'une quelconque des revendications précédentes, dans laquelle la silice est présente en une quantité comprise entre 0,01 % en poids et 5 % en poids par rapport au poids total de la composition ingestible.

6. Composition ingestible selon l'une quelconque des revendications précédentes, dans laquelle l'agent tensio-actif ingestible est un agent tensio-actif à base de polyéthylène, de polypropylène ou de polyoxyéthylène.

7. Composition ingestible selon la revendication 6, dans laquelle l'agent tensio-actif à base de polyéthylène est un polyéthylèneglycol.

8. Composition ingestible selon la revendication 7, dans laquelle le polyéthylèneglycol a un poids moléculaire compris entre 200 et 40 000, de préférence entre 200 et 1000.

9. Composition ingestible selon la revendication 6, dans laquelle l'agent tensio-actif à base de polyoxyéthylène est un ester d'acide gras de polyoxyéthylène-sorbitanne.

10. Composition ingestible selon la revendication 6, dans laquelle l'agent tensio-actif est un monostéarate de polyoxyéthylène ou un oxystéarate de glycérol-polyéthylèneglycol.

11. Composition ingestible selon l'une quelconque des revendications précédentes, dans laquelle l'agent tensio-actif ingestible est présent en une quantité comprise entre 0,01 % en poids et 5 % en poids par rapport au poids total de la composition ingestible.

12. Composition ingestible selon la revendication 11, dans laquelle l'agent tensio-actif ingestible est un polyéthylèneglycol et est présent en une quantité comprise entre 0,1 % en poids et 2 % en poids par rapport au poids total de la composition ingestible.

13. Composition ingestible selon la revendication 11, dans laquelle l'agent tensio-actif est un ester d'acide gras de polyoxyéthylène-sorbitanne et est présent en une quantité comprise entre 1 % en poids et 2 % en poids par rapport au poids total de la composition ingestible.

14. Procédé de fabrication d'une composition ingestible comprenant de l'ispaghula, de la silice colloïdale et un agent tensio-actif ingestible, le procédé comprenant l'étape consistant à mélanger l'ispaghula avec la silice colloïdale et l'agent tensio-actif ingestible ; de préférence sans utiliser d'alcool isopropylique ni, de préférence encore, aucun solvant ; et de préférence sans utiliser de polyvinylpyrrolidone ni, de préférence encore, aucun agent de granulation.
